# EUROPEAN PATENT APPLICATION

(11) **EP 4 851 287 A1**
(43) Date of publication of application: **09.12.2026**
(21) Application number: 25744622.9
(22) Date of filing: 22.01.2025
(51) Int. Cl.: C12N 15/113, C12N 9/22, C12N 15/90, A61K 48/00, A61K 38/46, A61P 9/12, A61P 9/10, A61P 9/04

(54) **GRNA TARGETING AGT GENE AND USE THEREOF**

(30) Priority: 22.01.2024 CN 202410087710
(71) Applicant: YOLTECH THERAPEUTICS CO., LTD., Minhang District, Shanghai 201109 (CN)
(72) Inventor: ZHANG, Haokun, Shanghai 201109 (CN); WU, Qianxuan, Shanghai 201109 (CN)
(74) Representative: CH Kilger Anwaltspartnerschaft mbB
(86) International application number: PCT/CN2025/074000
(87) International publication number: WO 2025/157184

(57) **Abstract**

The disclosure provides a gRNA targeting the AGT gene and uses thereof. Specifically, the present disclosure provides a gRNA targeting the exon-intron splicing junction regions of the AGT gene. The present disclosure has discovered for the first time that gRNAs designed to target the splicing junction regions of the AGT gene (e.g., the intron 1 acceptor, intron 2 donor and acceptor, and intron 4 donor and acceptor) can effectively inhibit AGT gene expression.

## Description

### Cross-Reference to Related Applications

This application claims the benefit of priority to Chinese Patent Application No. CN2024100877106, filed on January 22, 2024, entitled "gRNA Targeting AGT Gene and Use Thereof". The content of the aforementioned patent application, including any sequence listing and drawings thereof, is incorporated herein by reference in its entirety.

### Reference to Electronic Sequence Listing

The disclosure contains an electronic sequence listing, which is incorporated herein by reference in its entirety. Where a sequence is an RNA sequence, thymine (T) in said sequence shall be construed as uracil (U).

### TECHNICAL FIELD

The disclosure relates to the field of gene editing, and in particular, to gRNAs targeting the AGT gene and uses thereof.

### BACKGROUND

Hypertension is a major risk factor for ischemic heart disease, stroke, and chronic kidney disease, and is also the leading preventable cause of cardiovascular mortality worldwide. Although effective treatment regimens are available, nearly half of patients with hypertension fail to achieve guideline-recommended blood pressure targets, in part due to physicians' failure to initiate or intensify antihypertensive therapy and poor patient adherence to prescribed daily oral medications. Even when blood pressure appears well-managed based on intermittent office measurements, blood pressure control may remain suboptimal due to the significant variability of blood pressure over the diurnal cycle and over the long term.

The renin-angiotensin-aldosterone system (RAAS) plays a central role in blood pressure regulation. Angiotensinogen (AGT) has been demonstrated to be a therapeutic target for hypertension. Specific reduction of hepatic angiotensinogen messenger RNA (mRNA) levels, thereby decreasing angiotensinogen production, has been clinically shown to effectively lower blood pressure (Desai AS, Webb DJ, Taubel J, Casey S, Cheng Y, Robbie GJ, Foster D, Huang SA, Rhyee S, Sweetser MT, Bakris GL. Zilebesiran, an RNA Interference Therapeutic Agent for Hypertension. N Engl J Med. 2023 Jul 20;389(3):228-238. doi: 10.1056/NEJMoa2208391. PMID: 37467498.). As AGT is the sole precursor of all angiotensin peptides, inhibition of the RAAS via AGT gene knockout can theoretically limit compensatory angiotensin activation associated with angiotensin-converting enzyme inhibition or angiotensin receptor blockade, thereby achieving a superior blood pressure-lowering effect.

Currently, a small number of AGT small-molecule inhibitors are under clinical development. However, small-molecule inhibitors suffer from drawbacks including the need for long-term administration, tendency to develop drug resistance, and poor targeting specificity. It is generally recognized that compared with small-molecule chemical drugs, antibody drugs, recombinant proteins, and siRNA therapeutics, gene therapies (including CRISPR gene editing therapies) achieve durable and stable therapeutic effects through manipulation at the genetic level, reduce adverse reactions caused by long-term administration, offer convenient administration, and entail relatively lower long-term treatment costs. In addition, therapeutic approaches via editing regulatory elements in non-coding regions are relatively more likely to achieve a balance between therapeutic efficacy and safety.

Genome editing technology is a genetic engineering technique that uses engineered nucleases to modify specific positions in the genome of an organism. In particular, the CRISPR/Cas9 technology has garnered extensive attention from researchers due to its ease of operation, high efficiency, and broad applicability. With the development of CRISPR/Cas9 technology, single-base editing technologies established on this basis, including cytosine base editing systems and adenine base editing systems, are used to direct targeted base substitutions in target genes. Adenine base editing (ABE) technology primarily utilizes a fusion protein composed of a Cas protein and an adenosine deaminase. Guided by sgRNA, the fusion protein deaminates the target adenine (A) within the base editing activity window to form inosine (I), which is then gradually replaced by guanine (G) following DNA repair and replication, ultimately achieving a directional A-to-G substitution (A>G). Since its development, base editing technology has been successfully applied in a variety of organisms including plants and animals, owing to its advantages such as high efficiency, independence from DNA double-strand breaks, and no requirement for donor DNA.

The mechanism of gene knockout via ABE relies on the editing of splicing junctions in the target gene. Splicing junctions refer to the sequences flanking the cleavage and re-ligation sites. The junction at the 5' end of an intron is termed the donor site, and the junction at the 3' end is termed the acceptor site. In all structural genes (i.e., genes encoding polypeptides) in the nucleus, introns share the consensus sequence GU...AG at exon-intron junctions. Since ABE enables directional A-to-G substitutions (A>G) in the genome, it can disrupt splicing junctions. Following ABE-mediated gene editing, the GU...AG consensus sequence at splicing junctions is disrupted and converted to GC...AG or GU...GG, resulting in exon skipping or aberrant splicing during mRNA splicing. This in turn causes frameshift mutations and introduces premature stop codons, leading to mRNA degradation and ultimately achieving gene knockout.

Currently, there are no therapies targeting AGT gene knockout available on the market, and there remains a potential for unpredictable toxicity following AGT gene editing.

Citation or identification of any document in this disclosure is not an admission that such document is available as prior art to the disclosure.

### SUMMARY

In view of the foregoing problems, there is an urgent need for a gRNA targeting the AGT gene. Such gRNA can be useful in gene editing therapy to achieve a stable long-term therapeutic effect through genetic manipulation, reduce adverse reactions caused by long-term administration (e.g., drug resistance), and offer the advantages of convenient treatment with relatively lower cost in long-term treatment.

The human AGT gene is located on chromosome 1. To obtain gRNAs capable of efficiently targeting and editing the AGT gene, the inventors conducted investigations and experimental verification on the human AGT gene sequence, and identified gRNAs that specifically target the AGT gene.

In one aspect, the disclosure provides a gRNA that targets the splicing junction region of the AGT gene.

In some embodiments, the splicing junction region comprises the intron 1 acceptor, the intron 2 donor and acceptor, and the intron 4 donor and acceptor.

In some embodiments, the gRNA targets the GU···AG splicing junction within the splicing junction region of the AGT gene.

In some embodiments, the gRNA guides a base editor to target the human AGT gene and alter the AGT gene sequence, such that the AGT gene expression is down regulated by 1%, 2%, 5%, 10%, 15%, 20%, 30%, 40%, 50%, 60%, 70%, 80%, 85%, 90%, 95%, or 99%.

In some embodiments, the gRNA is capable of guiding a base editor to disrupt the splicing junctions of the AGT gene, thereby lowering the expression level of the AGT gene.

In some embodiments, the targeting sequence of the gRNA has an identity of 90%, 91%, 92%, 93%, 94%, 95%, 96%, 97%, 98%, 99%, or 100% to the sequence represented by any one of SEQ ID NOs: 1-7, or is the reverse complement of a sequence having an identity of 90%, 91%, 92%, 93%, 94%, 95%, 96%, 97%, 98%, 99%, or 100% to the sequence represented by any one of SEQ ID NOs: 1-7.

In some embodiments, the site targeted by the gRNA is located in the splicing junction region, and the target sequence targeted by the gRNA is obtained by deleting, adding, or substituting one or more nucleotides based on the sequence represented by any one of SEQ ID NOs: 1-7, resulting in a difference of for example not more than 1, 2, 3, 4, or 5 nucleotides from the sequence represented by any one of SEQ ID NOs: 1-7.

In some embodiments, the targeting sequence of the gRNA is represented by any one of SEQ ID NOs: 1-7, or is the reverse complement of the sequence represented by any one of SEQ ID NOs: 1-7.

In some embodiments, the gRNA can be chemically synthesized directly, or prepared by other methods, for example, by in vitro transcription (IVT).

In some embodiments, the gRNA comprises a targeting sequence that targets a target nucleic acid.

In some embodiments, the gRNA includes unmodified gRNA and modified gRNA. In some embodiments, the modified gRNA comprises a chemical modification of a nucleobase.

In some embodiments, the chemical modification comprises methylation modification, methoxy modification, fluorination modification, or phosphorothioate modification.

In some embodiments, the base editor is a base editor fused to a nuclease. In some embodiments, the nuclease comprises a Cas protein.

In some embodiments, the nuclease comprises a Type II Cas protein or a Type V Cas protein.

In some embodiments, the nuclease is Cas9, CasX, CasY, Cpf1, C2c1, C2c2, C2c3, Cas12a, Cas12b, Cas12g, Cas12h, Cas12i, Cas13b, Cas13c, Cas13d, Cas14, or Argonaute (Ago).

In some embodiments, the base editor comprises an adenine base editor (ABE). In some embodiments, the base editor comprises ABE8e.

In yet another aspect, the disclosure provides a nucleic acid encoding a gRNA of the disclosure, or encoding a precursor of a gRNA of the disclosure.

In yet another aspect, the disclosure provides a vector comprising a gRNA of the disclosure or a nucleic acid of the disclosure.

In some embodiments, the vector further comprises a nucleic acid encoding a base editor.

In some embodiments, the vector comprises one or more vectors, including:
a) a first regulatory element operably linked to said gRNA;
b) a second regulatory element operably linked to the base editor;
wherein components a) and b) are located on the same vector or on separate vectors.

In some embodiments, the vector is a vector for targeted editing of the AGT gene. In some embodiments, the vector comprises a plasmid or a viral vector.

In some embodiments, the viral vector is selected from the group consisting of adeno-associated virus (AAV), adenovirus, lentivirus, retrovirus, herpes virus, SV40, poxvirus, and combinations thereof.

In yet another aspect, the disclosure provides a gene editing system comprising a gRNA of the disclosure.

In some embodiments, the gene editing system further comprises a base editor.

In some embodiments, the base editor is a base editor fused to a nuclease. In some embodiments, the nuclease comprises a Cas protein.

In some embodiments, the nuclease comprises a Type II Cas protein or a Type V Cas protein.

In some embodiments, the nuclease comprises Cas9, CasX, CasY, Cpf1, C2c1, C2c2, C2c3, Cas12a, Cas12b, Cas12g, Cas12h, Cas12i, Cas13b, Cas13c, Cas13d, Cas14, or Argonaute (Ago).

In some embodiments, the base editor comprises an adenine base editor (ABE). In some embodiments, the base editor comprises ABE8e.

In some embodiments, the gene editing comprises in vivo gene editing and in vitro gene editing.

In yet another aspect, the disclosure provides a cell obtained by editing it with the gene editing system described herein.

In some embodiments, the cell is an in vitro cultured cell.

In some embodiments, the cell comprises a primary cell or a passaged cell.

In some embodiments, the cell comprises a mammalian cell.

In some embodiments, the cell comprises a human cell.

In some embodiments, the cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

In yet another aspect, the disclosure provides a composition comprising a gRNA component and a protein component, wherein the gRNA component comprises a gRNA of the disclosure, and the protein component comprises a base editor.

In some embodiments, the gRNA component is selected from a gRNA of the disclosure or a nucleic acid encoding said gRNA; the protein component is selected from a base editor or a nucleic acid encoding the base editor.

In some embodiments, the base editor is a base editor fused to a nuclease. In some embodiments, the nuclease comprises a Cas protein.

In some embodiments, the nuclease comprises a Type II Cas protein or a Type V Cas protein.

In some embodiments, the nuclease comprises Cas9, CasX, CasY, Cpf1, C2c1, C2c2, C2c3, Cas12a, Cas12b, Cas12g, Cas12h, Cas12i, Cas13b, Cas13c, Cas13d, Cas14, or Argonaute (Ago).

In some embodiments, the base editor comprises an adenine base editor. In some embodiments, the base editor comprises ABE8e.

In some embodiments, the composition comprises a pharmaceutical composition. In some embodiments, the pharmaceutical composition is in a liquid form.

In some embodiments, the dosage form of the pharmaceutical composition comprises an injection. In some embodiments, the dosage form of the pharmaceutical composition is an intravenous injection.

In yet another aspect, the disclosure provides a composition comprising:
the system of the disclosure or the cell of the disclosure; and a pharmaceutically acceptable carrier.

In some embodiments, the system or the cell accounts for 1 to 99 wt%, preferably 10 to 90 wt%, more preferably 30 to 70 wt% of the total weight of the composition.

In yet another aspect, the disclosure provides a delivery composition comprising an active ingredient and a delivery vehicle, wherein the active ingredient comprises a gRNA of the disclosure, a vector of the disclosure, or a system of the disclosure.

In some embodiments, the delivery composition further comprises a base editor.

In some embodiments, the delivery vehicle comprises lipid particles, sugar particles, metal particles, protein particles, liposomes, exosomes, microvesicles, gene guns, or viral vectors (e.g., replication-defective retroviruses, lentiviruses, adenoviruses, or adeno-associated viruses).

In yet another aspect, the disclosure provides a host cell comprising a gRNA of the disclosure, a nucleic acid of the disclosure, a vector of the disclosure, a system of the disclosure, a composition of the disclosure, or a delivery composition of the disclosure.

In some embodiments, the host cell comprises a mammalian cell. In some embodiments, the host cell comprises a human cell.

In some embodiments, the host cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

In yet another aspect, the disclosure provides a CRISPR complex comprising a gRNA of the disclosure, a base editor, and a target nucleic acid bound to said gRNA.

In yet another aspect, the disclosure provides a kit comprising: a first container, and an active ingredient or a medicament containing said active ingredient, wherein the active ingredient or the medicament is contained in the first container; the active ingredient comprises a gRNA of the disclosure, a nucleic acid of the disclosure, a vector of the disclosure, a system of the disclosure, a cell of the disclosure, a composition of the disclosure, a delivery composition of the disclosure, a host cell of the disclosure, or a CRISPR complex of the disclosure.

In some embodiments, the active ingredient or the medicament is a single-active ingredient preparation.

In some embodiments, the dosage form of the active ingredient or the medicament is an injection. In some embodiments, the dosage form of the active ingredient or the medicament is an intravenous injection.

In some embodiments, the kit further comprises instructions of administering the active ingredient or the medicament to a subject in need thereof, so as to (i) increase gene editing efficiency; and/or (ii) prevent and/or treat a disease.

In some embodiments, the disorder or disease comprises an angiotensinogen-related disease.

In some embodiments, the angiotensinogen-related disease is selected from the group consisting of hypertension, borderline hypertension, essential hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atheroma, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary aldosteronism and other mineralocorticoid excess states, sleep apnea, thyroid/parathyroid disorders, heart failure, myocardial infarction, angina pectoris, stroke, diabetes mellitus, nephropathy, renal failure, systemic sclerosis, intrauterine growth restriction (IUGR), and fetal growth restriction.

In some embodiments, the angiotensinogen-related disease is selected from the group consisting of hypertension, hypertensive heart disease, hypertensive nephropathy, pregnancy-associated hypertension, atheroma, arteriosclerosis, chronic kidney disease, glomerulosclerosis, aortic coarctation, aortic aneurysm, ventricular fibrosis, Cushing's syndrome and other glucocorticoid excess states (including chronic steroid therapy), pheochromocytoma, primary aldosteronism and other mineralocorticoid excess states, sleep apnea, thyroid/parathyroid disorders, heart failure, myocardial infarction, stroke, diabetes mellitus, renal failure, and systemic sclerosis.

In some embodiments, the angiotensinogen-related disease is pregnancy-associated hypertension (e.g., pregnancy-induced hypertension, preeclampsia, and eclampsia).

In some embodiments, the disorder or disease is hypertension.

In some embodiments, the subject of administration is a cell.

In some embodiments, the subject of administration is a human cell.

In some embodiments, the subject of administration comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

In some embodiments, the subject of administration is a human or a non-human mammal.

In some embodiments, the administration refers to contacting the active ingredient or the medicament with a human hepatocyte, or injecting the active ingredient or the medicament into a human body.

In yet another aspect, the disclosure provides method for gene editing a cell, comprising contacting said cell with a gRNA of the disclosure, a nucleic acid of the disclosure, a vector of the disclosure, a system of the disclosure, a composition of the disclosure, a delivery composition of the disclosure, a host cell of the disclosure, a CRISPR complex of the disclosure, or a kit of the disclosure.

In some embodiments, the contacting is performed in vitro.

In some embodiments, the cell is an in vitro cultured cell.

In some embodiments, the cell comprises a primary cell or a passaged cell.

In some embodiments, the cell comprises a mammalian cell. In some embodiments, the cell comprises a human cell.

In some embodiments, the cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

In yet another aspect, the disclosure provides a gene-editing kit, comprising a gRNA of the disclosure, a nucleic acid of the disclosure, a vector of the disclosure, a system of the disclosure, a composition of the disclosure, a delivery composition of the disclosure, a host cell of the disclosure, a CRISPR complex of the disclosure, or a kit of the disclosure described above.

In some embodiments, the gene-editing kit further comprises a label or instructions.

In some embodiments, the label or instructions show instructions of administering the gRNA of the disclosure, the nucleic acid of the disclosure, the vector of the disclosure, the system of the disclosure, the composition of the disclosure, the delivery composition of the disclosure, the host cell of the disclosure, the CRISPR complex of the disclosure, or the kit of the disclosure described above to a subject to be edited, so as to perform gene editing.

In some embodiments, the subject to be edited comprises a cell.

In some embodiments, the cell comprises a primary cell or a passaged cell.

In some embodiments, the cell comprises a mammalian cell.

In some embodiments, the cell comprises a human cell.

In some embodiments, the cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

In yet another aspect, the disclosure provides use of a gRNA of the disclosure, a nucleic acid of the disclosure, a vector of the disclosure, a system of the disclosure, a composition of the disclosure, a delivery composition of the disclosure, a host cell of the disclosure, a CRISPR complex of the disclosure, or a kit or gene-editing kit of the disclosure in gene editing, gene targeting, or gene cleavage.

In yet another aspect, the disclosure provides use of a gRNA of the disclosure, a nucleic acid of the disclosure, a vector of the disclosure, a system of the disclosure, a composition of the disclosure, a delivery composition of the disclosure, a host cell of the disclosure, a CRISPR complex of the disclosure, or a kit or gene-editing kit of the disclosure in the manufacture of a medicament for prevention and/or treatment of a disease.

In some embodiments, the disease is a disorder caused by a defect in a target sequence at a target locus (e.g., the AGT gene).

In some embodiments, the disorder or disease comprises an angiotensinogen-related disease.

In some embodiments, the angiotensinogen-related disease is selected from the group consisting of hypertension, borderline hypertension, essential hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atheroma, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary aldosteronism and other mineralocorticoid excess states, sleep apnea, thyroid/parathyroid disorders, heart failure, myocardial infarction, angina pectoris, stroke, diabetes mellitus, nephropathy, renal failure, systemic sclerosis, intrauterine growth restriction (IUGR), and fetal growth restriction.

In some embodiments, the angiotensinogen-related disease is selected from the group consisting of hypertension, hypertensive heart disease, hypertensive nephropathy, pregnancy-associated hypertension, atheroma, arteriosclerosis, chronic kidney disease, glomerulosclerosis, aortic coarctation, aortic aneurysm, ventricular fibrosis, Cushing's syndrome and other glucocorticoid excess states (including chronic steroid therapy), pheochromocytoma, primary aldosteronism and other mineralocorticoid excess states, sleep apnea, thyroid/parathyroid disorders, heart failure, myocardial infarction, stroke, diabetes mellitus, renal failure, and systemic sclerosis.

In some embodiments, the angiotensinogen-related disease is pregnancy-associated hypertension (e.g., pregnancy-induced hypertension, preeclampsia, and eclampsia).

In some embodiments, the disorder or disease is hypertension.

In yet another aspect, the disclosure provides a method for treating a disease, comprising administering to a subject an effective amount of the system of the disclosure, the cell of the disclosure, the composition of the disclosure, the delivery composition of the disclosure, the host cell of the disclosure, the CRISPR complex of the disclosure, the pharmaceutical kit of the disclosure, or the gene-editing kit of the disclosure.

In some embodiments, the administration comprises administration by injection.

In some embodiments, the subject is a human or a non-human mammal.

In some embodiments, the non-human mammal comprises rodents and primates, preferably mice, rats, rabbits, or monkeys.

In some embodiments, the disease is a disorder caused by a defect in a target sequence at a target locus (e.g., the AGT gene).

In some embodiments, the disorder or disease comprises an angiotensinogen-related disease.

In some embodiments, the angiotensinogen-related disease is selected from the group consisting of hypertension, borderline hypertension, essential hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atheroma, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary aldosteronism and other mineralocorticoid excess states, sleep apnea, thyroid/parathyroid disorders, heart failure, myocardial infarction, angina pectoris, stroke, diabetes mellitus, nephropathy, renal failure, systemic sclerosis, intrauterine growth restriction (IUGR), and fetal growth restriction.

In some embodiments, the angiotensinogen-related disease is selected from the group consisting of hypertension, hypertensive heart disease, hypertensive nephropathy, pregnancy-associated hypertension, atheroma, arteriosclerosis, chronic kidney disease, glomerulosclerosis, aortic coarctation, aortic aneurysm, ventricular fibrosis, Cushing's syndrome and other glucocorticoid excess states (including chronic steroid therapy), pheochromocytoma, primary aldosteronism and other mineralocorticoid excess states, sleep apnea, thyroid/parathyroid disorders, heart failure, myocardial infarction, stroke, diabetes mellitus, renal failure, and systemic sclerosis.

In some embodiments, the angiotensinogen-related disease is pregnancy-associated hypertension (e.g., pregnancy-induced hypertension, preeclampsia, and eclampsia).

In some embodiments, the disorder or disease is hypertension.

It is to be understood that within the scope of the disclosure, the above technical features of the disclosure and the technical features specifically described below (e.g., in the examples) may be combined with each other to form new or preferred technical solutions. Due to limited space, they are not exhaustively recited herein.

### BRIEF DESCRIPTION OF THE DRAWINGS

Figure 1 shows the genomic positions of different gRNAs (the images from left to right (along the direction of the arrow) correspond to the genome from the 5' end to the 3' end).
Figure 2 shows deep sequencing results of base editing efficiencies guided by different sgRNAs.
Figure 3 shows changes in AGT mRNA levels resulting from base editing guided by different sgRNAs.

### Detailed Description of Embodiments

Through extensive and in-depth research, the applicant of the disclosure has unexpectedly discovered for the first time that gRNAs designed to target the splicing junction regions of the AGT gene (such as the intron 1 acceptor, intron 2 donor/acceptor, and intron 4 donor/acceptor) can effectively inhibit AGT gene expression. Furthermore, the applicant has also found for the first time that the gRNAs of the disclosure designed for the GU-AG splice sites in the splicing junction regions can mediate very high gene editing efficiency, and hold potential application prospects for disease treatment.

### DEFINITIONS

"Splice site mutation" refers to a mutation that alters or abolishes the correct splicing of pre-mRNA sequences, resulting in a protein with an amino acid sequence different from that of the wild type. For example, during RNA splicing, one or more exons may be skipped, producing a protein lacking the amino acids encoded by the skipped exons. Alternatively, the reading frame may be altered due to incorrect splicing; one or more introns may be retained; additional splice donors or acceptors may be generated; splicing may initiate at other positions (e.g., within an intron); or alternative polyadenylation signals may be generated. Proper pre-mRNA splicing is a complex process that can be affected by various mutations in the nucleotide sequence of a GTR-encoding gene. In higher eukaryotes (such as plants), the major spliceosome processes introns that contain GU at the 5' splice site (donor site) and AG at the 3' splice site (acceptor site). Approximately 99% of splice sites in nuclear eukaryotic genes follow this GU-AG rule (or GT-AG rule; see Lewin, Genes VI, Oxford University Press, 1998, pp. 885-920, ISBN 0198577788).

The term "in vitro transcription (IVT)" refers to a technique for generating RNA via transcription in a cell-free in vitro system containing RNA polymerase, NTPs and other required components, using linear DNA as a template to mimic the in vivo transcription process.

The term "cell transfection" refers to a technique that introduces exogenous molecules such as DNA and RNA into eukaryotic cells with the aid of transfection reagents.

The term "base editor", also referred to as "nucleobase editor (NBE)", refers to an agent that binds to a polynucleotide and possesses nucleobase modification activity. In some embodiments, a base editor comprises a nucleobase modification polypeptide (e.g., a deaminase) and a polynucleotide-programmable nucleotide-binding domain (e.g., Cas9, Cas12) that binds to a guide polynucleotide (e.g., guide RNA). In some embodiments, the domain having base editing activity is capable of deaminating a base within a nucleic acid molecule. In some embodiments, the base editor is capable of deaminating one or more bases within a DNA molecule. In some embodiments, the base editor is capable of deaminating cytosine (C) or adenine (A) in DNA. In some embodiments, the base editor is a cytidine base editor (CBE). In some embodiments, the base editor is an adenine base editor (ABE). In some embodiments, the base editor comprises both an adenine base editor (ABE) and a cytidine base editor (CBE). In some embodiments, the base editor is a nuclease-dead Cas protein (e.g., dCas9, dCas12) fused to an adenosine deaminase.

The term "base editing activity" refers to the ability to chemically modify a base within a polynucleotide. In one embodiment, a first base is converted to a second base. In one embodiment, the base editing activity is cytidine deaminase activity, e.g., converting a target C·G base pair to T·A. In another embodiment, the base editing activity is adenosine or adenine deaminase activity, e.g., converting a target A·T base pair to G·C. In yet another embodiment, the base editing activity comprises both cytidine deaminase activity, e.g., converting a target C·G base pair to T·A, and adenosine or adenine deaminase activity, e.g., converting an A·T base pair to G·C.

The term "gRNA", also known as guide RNA, crRNA or guide sequence, generally comprises a scaffold sequence (e.g. a direct repeat sequence) and a guide sequence, or consists essentially of, or consists of, a scaffold sequence and a guide sequence. In certain instances, the guide sequence is any polynucleotide sequence that has sufficient complementarity to a target sequence to hybridize therewith and direct specific binding of a CRISPR/Cas complex to said target sequence. When optimally aligned, the degree of complementarity between the guide sequence and its corresponding target sequence is at least 50%, at least 60%, at least 70%, at least 80%, at least 90%, at least 95%, at least 99%, or 100% (complete complementarity).

### Gene Editing System

The disclosure provides a gene editing system comprising a gRNA of the disclosure, wherein said gRNA is capable of targeting a target sequence.

In some embodiments, the gene editing system further comprises a base editor.

In some embodiments, the target sequence is a splicing junction region of the AGT gene.

In some embodiments, the splicing junction region comprises the intron 1 acceptor, intron 2 donor/acceptor, and intron 4 donor/acceptor.

In some embodiments, the target sequence is a splice site within the splicing junction region of the AGT gene.

The gene editing system of the disclosure can significantly improve gene editing efficiency.

It will be appreciated by those skilled in the art that when base editors with different Cas proteins are employed, other conventional scaffold sequences may be selected for use with the targeting sequences or adjacent sequences described in the present examples, and editing of the target gene of the disclosure can still be achieved.

Among the gRNA sequences of the disclosure, SEQ ID NOs: 1-7 are designed based on the sequence context of the splice sites. By editing the targeted splice sites, they induce splice site mutations, interfere with the transcription process of the target gene, and thereby reduce target gene expression.

In one embodiment, the target sequence of said sgRNA is selected from any one of AGT-gRNA1, AGT-gRNA2, AGT-gRNA3, AGT-gRNA4, AGT-gRNA5, AGT-gRNA6, and AGT-gRNA7. The disclosure has confirmed through experiments that the above sequences achieve favorable editing effects.

### Pharmaceutical Compositions and Methods of Administration

In another aspect, the disclosure further provides a pharmaceutical composition comprising (a) a safe and effective amount of the gene editing system of the disclosure or the cells of the disclosure; and (b) a pharmaceutically acceptable carrier or excipient. The dosage of the gene editing system of the disclosure is typically 10 µg to 100 mg per dose, optionally 100 to 1000 µg per dose.

In one embodiment, the concentration of said cells in the pharmaceutical composition is 1×10³ to 1×10⁸ cells per kg body weight, preferably 1×10⁵ to 1×10⁷ cells per kg body weight. For the purposes of the disclosure, an effective dosage is about 0.01 mg/kg to 50 mg/kg body weight, preferably 0.05 mg/kg to 10 mg/kg body weight, of the gene editing system of the disclosure administered to an individual. Furthermore, the gene editing system of the disclosure may be used alone or in combination with other therapeutic agents (e.g., formulated in the same pharmaceutical composition).

The pharmaceutical compositions of the disclosure may be administered in the form of a cell therapeutic agent. For example, the gene editing system of the disclosure may be used to engineer cells, and the engineered cells may then be administered to a subject in need thereof via intravenous injection. In certain embodiments, the gene editing system of the disclosure may be used to engineer cells in vivo or isolated cells. In certain embodiments, the cells may be hepatocytes, and said engineering may comprise gene editing.

The pharmaceutical composition may further comprise a pharmaceutically acceptable carrier. The term "pharmaceutically acceptable carrier" refers to a carrier for the administration of a therapeutic agent. The term refers to pharmaceutical carriers that do not themselves induce the production of antibodies harmful to the individual receiving the composition, and exhibit no undue toxicity after administration. Such carriers are well known to those of ordinary skill in the art. A thorough discussion of pharmaceutically acceptable excipients can be found in Remington's Pharmaceutical Sciences (Mack Pub. Co., N.J. 1991). Such carriers include, but are not limited to, saline, buffer, glucose, water, glycerol, ethanol, adjuvants, and combinations thereof.

Pharmaceutically acceptable carriers in the therapeutic compositions may contain liquids such as water, saline, glycerol, and ethanol. In addition, auxiliary substances such as wetting or emulsifying agents, pH buffering agents, and the like may also be present in these carriers.

Typically, the therapeutic compositions may be prepared as injectables, for example, liquid solutions or suspensions; solid forms suitable for dissolution or suspension in a liquid vehicle prior to injection may also be prepared. Once formulated, the compositions of the disclosure may be administered via conventional routes, including, but not limited to, intramuscular, intravenous, subcutaneous, intradermal, or topical administration. The subject to be prevented or treated may be an animal; in particular, a human.

When the pharmaceutical composition of the disclosure is used in actual clinical treatment, various dosage forms may be adopted depending on the application scenario. Intravenous injection is preferred. These pharmaceutical compositions may be formulated by mixing, dilution, or dissolution according to conventional methods, with the occasional addition of suitable pharmaceutical additives such as excipients, disintegrants, binders, lubricants, diluents, buffering agents, isotonic agents, preservatives, wetting agents, emulsifiers, dispersing agents, stabilizers, and solubilizers. The formulation process may be performed in a conventional manner according to the dosage form.

The pharmaceutical compositions of the disclosure may also be administered in the form of sustained-release formulations. For example, the gene editing system of the disclosure may be incorporated into pellets or microcapsules with a sustained-release polymer as a carrier, which are then surgically implanted into the tissue to be treated. Examples of sustained-release polymers include ethylene-vinyl acetate copolymer, polyhydroxymethacrylate, polyacrylamide, polyvinylpyrrolidone, methylcellulose, polylactic acid polymers, lactic acid-glycolic acid copolymers, and the like. Preferred examples are biodegradable polymers such as polylactic acid polymers and lactic acid-glycolic acid copolymers.

In one embodiment, the pharmaceutical composition may comprise buffers such as neutral buffered saline, sulfate buffered saline, and the like; carbohydrates such as glucose, mannose, sucrose, dextran, or mannitol; proteins; polypeptides or amino acids such as glycine; antioxidants; chelating agents such as EDTA or glutathione; adjuvants (e.g., aluminum hydroxide); and preservatives. The pharmaceutical composition of the disclosure is preferably formulated for intravenous administration.

When the pharmaceutical composition of the disclosure is used in actual treatment, the dosage of the gene editing system of the disclosure as the active ingredient may be reasonably determined according to the body weight, age, sex, and severity of symptoms of each patient to be treated.

The main advantages of the disclosure include:
(1) It is discovered for the first time that gRNAs designed to target the splicing junction regions of the AGT gene (intron 1 acceptor, intron 2 donor/acceptor, and intron 4 donor/acceptor) can effectively inhibit AGT gene expression. All gRNAs designed for the GU-AG splice sites in the splicing junction regions exhibit excellent gene editing efficiency, among which 5 gRNAs have exceptionally high gene editing efficiency. The disclosure holds promising application potential for the treatment of diseases.
(2) The disclosure provides a gRNA targeting the AGT gene, which can be applied in CRISPR gene editing therapy. It achieves durable and stable therapeutic effects through genetic manipulation, reduces adverse reactions caused by long-term administration (e.g., drug resistance), and offers the advantages of convenient treatment and relatively lower long-term treatment costs.
(3) The base editing approach does not generate double-strand breaks or cause random insertion of large gene fragments, resulting in superior therapeutic efficacy and improved safety.

The following specific examples are provided to further illustrate certain embodiments of the disclosure. It is to be understood that these examples are only intended to illustrate certain embodiments of the disclosure and are not intended to limit the scope of the invention. Experimental methods in the following examples without specified conditions are generally performed under conventional conditions, such as those described in Sambrook et al., Molecular Cloning: A Laboratory Manual (New York: Cold Spring Harbor Laboratory Press, 1989), or under conditions recommended by the manufacturer. Unless otherwise specified, percentages and parts are percentages by weight and parts by weight. Unless otherwise specified, the cell lines listed in the context of the disclosure are cultured according to conventional techniques in the art. Unless otherwise defined, all technical and scientific terms used herein have the same meaning as commonly understood by one of ordinary skill in the art to which the disclosure pertains. The terminology used in the specification herein is for the purpose of describing particular embodiments only and is not intended to limit the invention. As used herein, the term "and/or" includes any and all combinations of one or more of the associated listed items.

Unless otherwise specified, materials and reagents used in the examples are commercially available products.

### Example 1 Chemical Synthesis of sgRNAs Targeting the Target Sequences

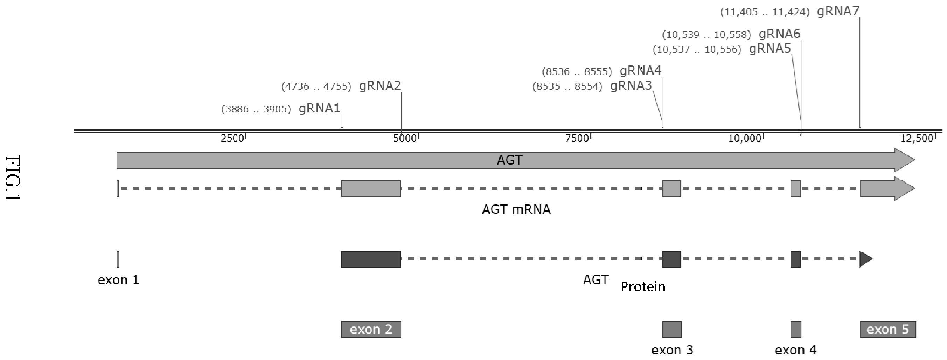

(1) The AGT gene sequence was retrieved from the NCBI database (GenBank, ID 8317). Based on the splicing junction regions of the human AGT gene, corresponding sgRNA targeting sequences were designed. The GU-AG splice sites capable of disrupting the AGT gene were identified, and a total of 7 sgRNA targeting sequences were designed, with their genomic positions shown in Figure 1. The specific design of the sgRNA targeting sequences is presented in Table 1.

**Table 1 Target sequences and editing sites of individual AGT-gRNAs**

| sgRNA Name | sgRNA Target Sequence | Targeting Site |
|---|---|---|
| AGT-gRNA1 | TTTCAGGGTaTGCGGAAGCG (SEQ ID NO:1) | Intron 1 acceptor |
| AGT-gRNA2 | CTTaCCTTGGAAGTGGACGT (SEQ ID NO:2) | Intron 2 donor |
| AGT-gRNA3 | TGGATTTCaGGGAAGATGAA (SEQ ID NO:3) | Intron 2 acceptor |
| AGT-gRNA4 | GGATTTCaGGGAAGATGAAG (SEQ ID NO:4) | Intron 2 acceptor |
| AGT-gRNA5 | ATaCCTCCCCCACCCTGATG (SEQ ID NO:5) | Intron 4 donor |
| AGT-gRNA6 | ACATaCCTCCCCCACCCTGA (SEQ ID NO:6) | Intron 4 donor |
| AGT-gRNA7 | CTGCTTTGCaGGTGCTGAAC (SEQ ID NO:7) | Intron 4 acceptor |

The scaffold sequence employed for the sgRNAs in Table 1 above is set forth below:

GTTTTAGAGCTAGAAATAGCAAGTTAAAATAAGGCTAGTCCGTTATCAACTT
GAAAAAGTGGCACCGAGTCGGTGC (SEQ ID NO: 17)

Each sgRNA is structured as a targeting sequence followed by a scaffold sequence in the 5' to 3' direction. For example, the sequence of AGT-gRNA1 is set forth below:

TTTCAGGGTaTGCGGAAGCGGTTTTAGAGCTAGAAATAGCAAGTTAAAATA
AGGCTAGTCCGTTATCAACTTGAAAAAGTGGCACCGAGTCGGTGC (SEQ ID
NO: 8)

The sgRNA sequences were chemically synthesized by GenScript Biotech Corp. Three nucleotides at both the 5' end and the 3' end of each sgRNA are modified with phosphorothioate linkages and methoxy modifications.

### Example 2 Synthesis of ABE8e mRNA

ABE8e mRNA was synthesized via the in vitro transcription (IVT) method. The detailed procedures are as follows:
(1) The ABE8e plasmid was purchased from Addgene (Plasmid #138489). It was then digested with BspQI restriction endonuclease (purchased from NEB) and linearized to serve as the template DNA for in vitro transcription. A PCR tube was picked up with forceps, placed on a tube rack, and labeled accordingly. Each reaction component was added sequentially with a corresponding pipette per Table 2, and mixed thoroughly by gentle pipetting.

**Table 2 Components for in vitro transcription (IVT) of ABE8e mRNA**

| Component | Volume Added |
|---|---|
| 10x Transcription Buffer(NEB) | 1 µl |
| NEB:A/C/G(NEB) | 1/1/1 µl |
| Trilink:pseudoU(Trilink) | 1 µl |
| Trilink:CapAG(Trilink) | 0.8 µl |
| NEB T7 enzyme mix (NEB) | 2 µl |
| Template DNA | 500 ng |
| Total volume | 20 µl (made up to final volume with RNase-free H₂O) |

(2) The PCR tube was capped and incubated in a PCR thermocycler at 37 °C for 2 h.
(3) After incubation, the PCR tube was removed from the thermocycler. A clean ice box was half-filled with ice, and DNase I (purchased from NEB, Cat. No. M0303S) was taken out from a -20 °C freezer and transferred together to a biosafety cabinet in the cell culture room. First, the sample tube was sprayed with RNaseZap^{TM} RNase Decontamination Solution (Invitrogen, Cat. No. AM9780), wiped clean with a clean paper towel, and placed on a PCR tube rack in the biosafety cabinet. Subsequently, 1 µL of DNase I (purchased from Takara Bio) was added to the reaction tube, mixed by gentle agitation, and incubated in the PCR thermocycler at 37 °C for 15 min.
(4) Upon completion of the reaction, the samples were transferred to the biosafety cabinet. First, a spin column from the RNeasy Mini Kit (purchased from Qiagen) was placed on a tube rack and the cap was opened. A clean 1.5 mL microcentrifuge tube was then placed on the tube rack. The reaction solution was aspirated into the tube using a 100 µL pipette, followed by addition of 350 µL of RLT Buffer and 250 µL of absolute ethanol into the tube. After gentle mixing with a 1 mL pipette, the mixture was loaded onto the spin column prepared above. The column was capped, transferred to a centrifuge, and centrifuged at 12,000 rpm for 30 sec.
(5) After centrifugation, the spin column was returned to the biosafety cabinet. The flow-through was aspirated with a 1 mL pipette, and the spin column was placed back into the collection tube. With the cap opened, the volume of the 1 mL pipette was adjusted to 500 µL, and 500 µL of RPE Buffer was aspirated and added onto the spin column. The column was capped, transferred to the centrifuge, and centrifuged at 12,000 rpm for 30 sec.
(6) After centrifugation, the spin column was returned to the biosafety cabinet. The flow-through was aspirated, and the spin column was placed back into the collection tube. With the cap opened, the volume of the 1 mL pipette was adjusted to 500 µL, and 500 µL of RPE Buffer was aspirated and added onto the spin column. The column was capped, transferred to the centrifuge, and centrifuged at 12,000 rpm for 2 min.
(7) After centrifugation, the spin column was returned to the biosafety cabinet. A clean collection tube was taken out, and the spin column was transferred to the clean collection tube, followed by an additional centrifugation at 12,000 rpm for 2 min.
(8) After centrifugation, the spin column was returned to the biosafety cabinet. A clean 1.5 mL microcentrifuge tube was taken out, and the spin column was transferred into the clean microcentrifuge tube. The cap was opened, and the assembly was left to stand on the tube rack for 2 min. The volume of a 100 µL pipette was adjusted to 50 µL, and 50 µL of nuclease-free water was aspirated and added onto the center of the spin column membrane. The tube was capped, left to stand on the tube rack for 1 min, and then centrifuged at 12,000 rpm for 1 min.
(9) After centrifugation, the spin column was returned to the biosafety cabinet. Using the same 50 µL pipette, another 50 µL of nuclease-free water was aspirated and added onto the center of the spin column membrane. The tube was capped, left to stand on the tube rack for 2 min, and centrifuged at 12,000 rpm for 1 min.
(10) After centrifugation, the spin column was returned to the biosafety cabinet. A clean PCR tube and a microcentrifuge tube were taken out and placed on the tube rack. All the eluate was collected into the prepared 1.5 mL microcentrifuge tube. Then, 3 µL of the sample was aspirated with a 10 µL pipette and transferred to the PCR tube for subsequent concentration measurement. The sample name and date were labeled, and both tubes were placed on ice.
(11) The concentration was marked on the tube containing the mRNA sample. The tube was sealed with Parafilm and stored at -80 °C. As measured by Nanodrop, the concentration of the obtained ABE8e mRNA was 1542 ng/µL.

### Example 3 Detection of Base Editing Efficiency at the AGT Target Gene Locus 1. Cell Culture

The Human Protein Atlas (https://www.proteinatlas.org/), a public database for human genome expression profiling, shows that the AGT gene is highly expressed in the HepG2 cell line (purchased from the Cell Bank of the Chinese Academy of Sciences, Cat. No. SCSP-510). Therefore, this cell line was selected as the subject for base editing.

### 1.1 Pre-experiment Preparation

(1) Opti-MEM^{TM} I Reduced Serum Medium (Gibco, Cat. No. 31985070) was equilibrated at room temperature for 30 min prior to use.
(2) The work surface of the biosafety cabinet was wiped and disinfected with 75% alcohol cotton, followed by UV irradiation for 30 min.

### 1.2 Preparation of Culture Medium

### (1) Medium formulation

To 500 mL of high-glucose DMEM (purchased from Gibco) were added 50 mL of FBS and 5 mL of penicillin-streptomycin solution (Gibco, Cat. No. 15140122).

(2) The prepared medium was filtered through a sterile filter unit, labeled on the bottle body, and stored at 4 °C.

### 1.3 Procedures

(1) After the airflow in the biosafety cabinet stabilized, the work surface was wiped with alcohol gauze, and required consumables and reagents were placed into the cabinet (all items were wiped and disinfected with alcohol gauze when passing through the workbench opening).
(2) The cell culture dish was removed from the incubator and observed under an inverted microscope for cell growth status. Cell passaging was performed when cell confluency reached 80-90%.
(3) The dish was taken out, and the culture supernatant was discarded from the dish. For a 100 mm culture dish, 3 mL of DPBS was added to cover the dish bottom for washing, and then discarded.
(4) 1 mL of 0.25% trypsin-EDTA was added to each 100 mm culture dish, and the dish was placed in a 37 °C incubator for digestion for 5 min. The dish was gently swirled to facilitate cell detachment.
(5) 3 mL of complete medium was added to neutralize the digestion. Cells were collected into a centrifuge tube, resuspended by gentle pipetting, and the total volume was recorded.
(6) 10 µL of cell suspension was taken out, into which 10 µL of AO/PI (purchased from Beijing Biolab Technology Co., Ltd., Cat. No. HR0462) was added and mixed by pipetting. The mixture was transferred to a cell counting slide, and cell counting was performed with an automated cell counter to calculate the total cell number.
(7) Centrifugation was performed at 400 g for 5 min at room temperature with balanced sample loading.
(8) After centrifugation, the supernatant was discarded. An appropriate volume of culture medium was added according to the total cell number to adjust the cell concentration to 2×10⁷ cells/mL. Cells were thoroughly resuspended by repeated pipetting, and 1 mL of cell suspension was seeded into a new 100 mm culture dish.
(9) The culture dish was homogenized by the cross-mixing method, placed in the incubator, and the seeding time was recorded.
(10) Reagents and consumables in the biosafety cabinet were sealed and removed. The work surface was wiped and disinfected with alcohol, and the UV disinfection lamp was turned on.

### 2. Cell Transfection

(1) HepG2 cells at 80-90% confluency were passaged and counted, then seeded into a 48-well plate at a density of 4×10⁴ cells per well. Cell density was examined 24 h later, and mRNA transfection was performed when cells reached 70% confluency.
(2) Transfection was carried out with calculated amounts of RNA and Lipofectamine MessengerMax per well of the 48-well plate. Three RNA dosage gradients were set: 100 ng, 300 ng, and 500 ng. The weight ratio of ABE mRNA to sgRNA was 1:1. Taking a total RNA transfection dosage of 500 ng as an example: 500 ng of total RNA (250 ng of sgRNA + 250 ng of ABE8e mRNA) was used with 1.5 µL of Lipofectamine MessengerMax (Invitrogen, Cat. No. LMRNA003).
   Two 1.5 mL microcentrifuge tubes were prepared, with 20 µL of Opti-MEM medium added to each. To one tube, 250 ng of sgRNA and 250 ng of ABE8e mRNA were added; to the other tube, 1.5 µL of Lipofectamine MessengerMax was added. The contents of each tube were mixed and left to stand at room temperature for 10 min.
(3) The reagents from the two microcentrifuge tubes were combined and left to stand at room temperature for 5 min. The mRNA-transfection reagent complexes were then added to the cells for transfection, with an editing duration of 72 h.

### 3. Genomic DNA Extraction

Genomic DNA of HepG2 cells was extracted using a Blood/Cell/Tissue Genomic DNA Extraction Kit (TIANGEN, Cat. No. DP304).
(1) Cells were resuspended in 200 µL of Buffer GA.
(2) 20 µL of Proteinase K was added and mixed thoroughly.
(3) 200 µL of Buffer GB was added, mixed thoroughly by inverting the tube, and incubated at 70 °C for 10 min until the solution became clear. The tube was briefly centrifuged to remove droplets from the inner wall of the tube cap.
(4) 200 µL of absolute ethanol was added and mixed thoroughly by vortexing for 15 s. Flocculent precipitates may form at this step. The tube was briefly centrifuged to remove droplets from the inner wall of the tube cap.
(5) The solution and flocculent precipitates obtained from the previous step were transferred to a CB3 spin column (placed in a collection tube). The column was centrifuged at 12,000 rpm for 30 s, the flow-through was discarded, and the CB3 spin column was placed back into the collection tube.
(6) 500 µL of Buffer GD (check that absolute ethanol has been added prior to use) was added to the CB3 spin column. The column was centrifuged at 12,000 rpm for 30 s, the flow-through was discarded, and the CB3 spin column was placed back into the collection tube.
(7) 600 µL of Buffer PW (check that absolute ethanol has been added prior to use) was added to the CB3 spin column. The column was centrifuged at 12,000 rpm for 30 s, the flow-through was discarded, and the CB3 spin column was placed back into the collection tube.
(8) Step (7) was repeated once.
(9) The column was centrifuged at 12,000 rpm for 2 min, and the flow-through was discarded. The CB3 spin column was left at room temperature for several minutes to thoroughly air-dry residual wash solution from the adsorption matrix.
(10) The CB3 spin column was transferred to a clean centrifuge tube. 50-200 µL of nuclease-free water was added dropwise to the center of the adsorption membrane, and the column was left to stand at room temperature for 2-5 min. The column was centrifuged at 12,000 rpm for 2 min, and the eluate was collected in the centrifuge tube.
(11) DNA concentration was measured using a NanoDrop^{™} Lite Spectrophotometer (Thermo Scientific).

### 4. Determination of Base Editing Efficiency

(1) Detection primers for the target sequence loci of each target gene are listed in Table 3. All primers were synthesized by Sangon Biotech (Shanghai) Co., Ltd.

**Table 3 Detection primers for target sequence loci**

| Primer Name | Primer Sequence (5'-3') |
|---|---|
| AGTsg1 deepseq-F | ggagtgagtacggtgtgcAGTAGGACCTGAAGAGAAAATTGC (SEQ ID NO:9) |
| AGTsg1 deepseq-R | gagttggatgctggatggTCCCCTCATCCCTGCTAAAT (SEQ ID NO:10) |
| AGTsg2 deepseq-F | ggagtgagtacggtgtgcAGTACAGCTGGCAGAAAGTGTT (SEQ ID NO:11) |
| AGTsg2 deepseq-R | gagttggatgctggatggCTTACTTTCACTGCAGGGCTC (SEQ ID NO:12) |
| AGTsg3-5 deepseq-F | ggagtgagtacggtgtgcACACCAGGATTCAGAGCACC (SEQ ID NO:13) |
| AGTsg3-5 deepseq-R | gagttggatgctggatggAATGCCACACTGCAAGAACTG (SEQ ID NO:14) |
| AGTsg6 deepseq-F | ggagtgagtacggtgtgcTGGGTCTGGGCATCTGAAA (SEQ ID NO:15) |
| AGTsg6 deepseq-R | gagttggatgctggatggATATCGTCCACTAAGCAAA (SEQ ID NO:16) |

### (2) PCR reaction system and procedure

The forward and reverse detection primers are listed in Table 3. For all PCR reactions, the target fragments were amplified using the high-fidelity DNA polymerase KOD-Plus-Neo (purchased from TOYOBO, Cat. No. KOD-401).

The amplification system was formulated as follows: 5 µL of 10× PCR Buffer for KOD-Plus-Neo, 5 µL of dNTPs, 3 µL of Mg²⁺, 1 µL of KOD-Plus-Neo, 1 µL each of the forward and reverse detection primers for target locus, 50 ng of DNA template, and ddH₂O to make up the total volume to 50 µL.

The PCR reaction program was set as follows: initial denaturation at 95 °C for 5 min; 35 cycles of 95 °C for 10 s, 60 °C for 10 s, and 68 °C for 10 s; final extension at 68 °C for 5 min; and hold at 4 °C.

The resulting PCR products were sent to Sangon Biotech (Shanghai) Co., Ltd. for deep sequencing.

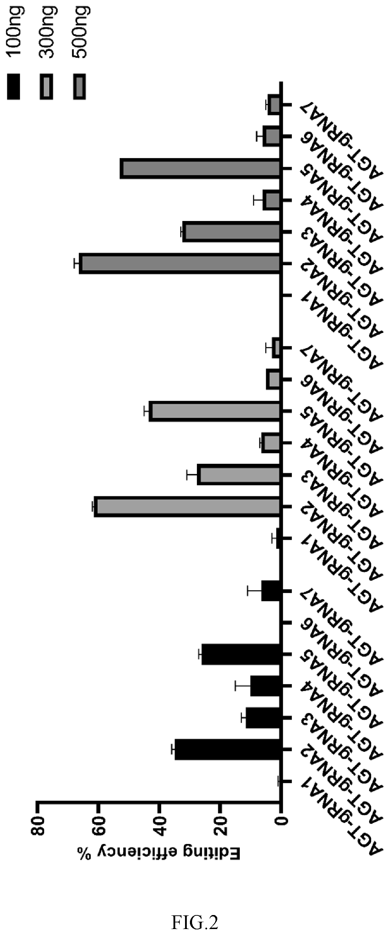

The sequencing results are presented in Figure 2, and demonstrate the following:
At a transfection dosage of 100 ng, the base editing efficiency mediated by AGT-gRNA1 was 0.5%, by AGT-gRNA2 was 35.0%, by AGT-gRNA3 was 11.5%, by AGT-gRNA4 was 10.0%, by AGT-gRNA5 was 26.0%, by AGT-gRNA6 was 0.0%, and by AGT-gRNA7 was 6.5%.

At a transfection dosage of 300 ng, the base editing efficiency mediated by AGT-gRNA1 was 1.5%, by AGT-gRNA2 was 61.5%, by AGT-gRNA3 was 27.5%, by AGT-gRNA4 was 6.5%, by AGT-gRNA5 was 43.5%, by AGT-gRNA6 was 5.0%, and by AGT-gRNA7 was 3.0%.

At a transfection dosage of 500 ng, the base editing efficiency mediated by AGT-gRNA1 was 0.0%, by AGT-gRNA2 was 66.5%, by AGT-gRNA3 was 32.5%, by AGT-gRNA4 was 6.0%, by AGT-gRNA5 was 53.0%, by AGT-gRNA6 was 6.0%, and by AGT-gRNA7 was 4.5%.

It can be concluded that gRNA2 and gRNA5 mediate relatively high base editing efficiency, with gRNA2 in particular achieving the highest, whereas gRNA1 and gRNA6 exhibit low editing efficiency and barely exert a mediating effect.

### Example 4 Detection of mRNA Transcription Levels Following AGT Target Gene Editing

### 1. Total RNA Extraction

Total RNA was extracted from HepG2 cells transfected with 300 ng of RNA using the Trizol method.
(1) Cells were collected by centrifugation, and 1 mL of Trizol was added, followed by repeated mixing.
(2) The homogenized sample was left to stand at room temperature (15-30 °C) for 5 minutes to allow complete dissociation of nucleoprotein complexes.
(3) 0.2 mL of chloroform was added per 1 mL of Trizol, followed by vigorous vortexing for 15 seconds and leaving it to stand at room temperature for 10 minutes.
(4) The sample was centrifuged at 12,000 × g for 15 minutes at 4 °C. The sample separated into three layers: a red organic phase at the bottom, a colorless aqueous phase at the top, and an interphase. RNA was predominantly present in the aqueous phase, which accounted for approximately 60% of the volume of the Trizol reagent used. The upper aqueous phase was transferred to a new microcentrifuge tube.
(5) An equal volume of isopropanol (approximately 500 µL) was added to the upper aqueous phase (approximately 500 µL) to precipitate RNA from the aqueous phase. After addition of isopropanol, the tube was mixed by inversion and left to stand at room temperature for 10 minutes.
(6) The sample was centrifuged at 12,000 × g for 10 minutes at 4 °C. No RNA precipitate was visible prior to centrifugation. After centrifugation, a gelatinous precipitate appeared on the side and bottom of the tube. The supernatant was removed.
(7) The RNA precipitate was washed with 1 mL of 75% ethanol and centrifuged at 12,000 × g for 5 minutes at 4 °C, after which the supernatant was discarded.
(8) The RNA precipitate was air-dried at room temperature for approximately 5-10 minutes. Excessive drying will significantly reduce the solubility of RNA.
(9) 50 µL of nuclease-free water was added and mixed. The concentration was measured by Nanodrop, and the RNA was stored at -80 °C for subsequent use.

### 2. cDNA Synthesis and Real-time PCR (qPCR)

(1) The reverse transcription system (10 µL) was formulated as follows:
   5 µL of 2× Hifair^{®} II SuperMix plus, 500 ng of total RNA, and RNase-free H₂O to make up the remaining volume.
(2) The RT-PCR program was set as follows:
   25 °C for 5 min, 42 °C for 30 min, and 85 °C for 5 min.

The resulting cDNA product was stored at -20 °C for subsequent use.

### 3. Real-time PCR Reaction

(1) 10 µL of the obtained cDNA was diluted with 40 µL of DNase/RNase-free water (i.e., 5-fold dilution of cDNA) and used as the template for subsequent Real-time qPCR.

The Real-time PCR reaction system (20 µL) was formulated as follows:
10 µL of Hieff^{®} qPCR SYBR Green Master Mix, 0.5 µL each of forward and reverse primers, 1 µL of Real-time qPCR template, and sterile ultrapure water to make up the remaining volume.

The Human AGT qPCR Primer Pair was provided by Beyotime Biotech Inc. (Shanghai), Cat. No. QH01141S.

The Real-time PCR reaction program was set as follows:
initial denaturation at 95 °C for 10 min; 40 cycles of denaturation at 95 °C for 15 s and annealing/extension at 60 °C for 30 s. Melting curves were generated using the instrument default settings, and mRNA expression levels were analyzed using the 2^{-ΔΔCT} method. The untreated group was selected as the control group; the NT group served as negative control 1 (i.e., the non-targeting group), and the AAVS1 group served as negative control 2 (i.e., the AAVS1 locus editing group).

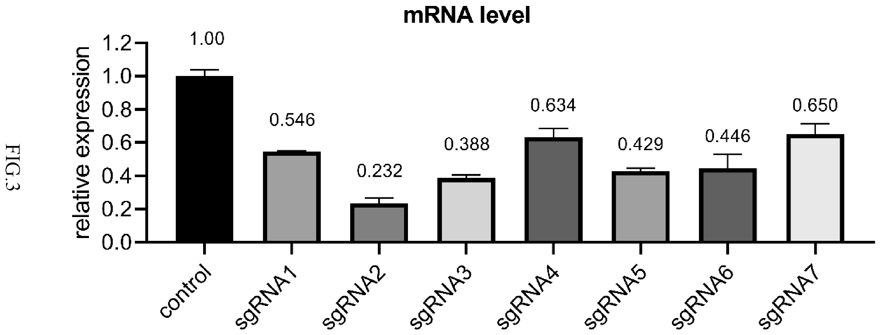

The mRNA expression levels are shown in Figure 3. Relative to the control group, editing mediated by AGT-gRNA1 reduced AGT mRNA expression by 45.4%; editing mediated by AGT-gRNA2 knocked down AGT mRNA expression by 76.8%; editing mediated by AGT-gRNA3 knocked down AGT mRNA expression by 61.2%; editing mediated by AGT-gRNA4 knocked down AGT mRNA expression by 36.6%; editing mediated by AGT-gRNA5 knocked down AGT mRNA expression by 57.1%; editing mediated by AGT-gRNA6 reduced AGT mRNA expression by 55.4%; and editing mediated by AGT-gRNA7 reduced AGT mRNA expression by 35.0%.

The above results demonstrate that AGT-gRNA2 of the disclosure can significantly knock down AGT mRNA expression levels, while other AGT-gRNAs also knock down AGT mRNA expression to varying degrees, such as AGT-gRNA3, AGT-gRNA5, and AGT-gRNA6. Notably, although AGT-gRNA1 mediates very low editing efficiency targeting the AGT gene, it still knocked down nearly 50% of AGT mRNA expression, indicating that AGT-gRNA1 still holds application prospects.

Taken together, the use of ABE (adenine base editor, e.g., ABE8e) in combination with the gRNAs of the disclosure can effectively inhibit AGT gene expression, and holds potential therapeutic application prospects.

All documents mentioned in the disclosure are incorporated herein by reference in their entirety, as if each individual document was specifically and individually indicated to be incorporated by reference. Furthermore, it should be understood that after reading the above teachings of the disclosure, those skilled in the art may make various changes or modifications to the disclosure, and such equivalent forms likewise fall within the scope defined by the appended claims of this application.

## Claims

1. A gRNA, wherein said gRNA targets a splicing junction region of the AGT gene;
optionally, said splicing junction region comprises the intron 1 acceptor, the intron 2 donor and acceptor, and the intron 4 donor and acceptor;
optionally, said gRNA targets a GU···AG splicing junction within the splicing junction region of the AGT gene;
optionally, said gRNA guides a base editor to target the human AGT gene and alter the AGT gene sequence, such that the AGT gene expression is downregulated by 1%, 2%, 5%, 10%, 15%, 20%, 30%, 40%, 50%, 60%, 70%, 80%, 85%, 90%, 95%, or 99%;
optionally, said gRNA is capable of guiding a base editor to disrupt splicing junctions of the AGT gene, thereby lowering the expression level of the AGT gene;
optionally, the targeting sequence of said gRNA has an identity of 90%, 91%, 92%, 93%, 94%, 95%, 96%, 97%, 98%, 99%, or 100% to the sequence represented by any one of SEQ ID NOs: 1-7, or is the reverse complement of a sequence having an identity of 90%, 91%, 92%, 93%, 94%, 95%, 96%, 97%, 98%, 99%, or 100% to the sequence represented by any one of SEQ ID NOs: 1-7;
optionally, the site targeted by said gRNA is located in the splicing junction region, and the target sequence targeted by said gRNA is obtained by deleting, adding, or substituting one or more nucleotides based on the sequence represented by any one of SEQ ID NOs: 1-7, resulting in a difference of for example not more than 1, 2, 3, 4, or 5 nucleotides from the sequence represented by any one of SEQ ID NOs: 1-7;
optionally, the targeting sequence of said gRNA is represented by any one of SEQ ID NOs: 1-7, or is the reverse complement of the sequence represented by any one of SEQ ID NOs: 1-7;
optionally, said gRNA can be chemically synthesized directly, or prepared by other methods, for example, by in vitro transcription (IVT);
optionally, said gRNA comprises a targeting sequence that targets a target nucleic acid;
optionally, said gRNA includes unmodified gRNA and modified gRNA;
optionally, said modified gRNA comprises a chemical modification of a nucleobase;
optionally, said chemical modification comprises methylation modification, methoxy modification, fluorination modification, or phosphorothioate modification;
optionally, said base editor is a base editor fused to a nuclease;
optionally, said nuclease comprises a Cas protein;
optionally, said nuclease comprises a Type II Cas protein or a Type V Cas protein;
optionally, said nuclease is Cas9, CasX, CasY, Cpf1, C2c1, C2c2, C2c3, Cas12a, Cas12b, Cas12g, Cas12h, Cas12i, Cas13b, Cas13c, Cas13d, Cas14, or Argonaute (Ago);
optionally, said base editor comprises an adenine base editor (ABE);
optionally, said base editor comprises ABE8e.

2. A nucleic acid encoding the gRNA of claim 1, or encoding a precursor of the gRNA of claim 1.

3. A vector, wherein said vector comprises the gRNA of claim 1 or the nucleic acid of claim 2;
optionally, said vector further comprises a nucleic acid encoding a base editor;
optionally, said vector comprises one or more vectors, including:
a) a first regulatory element operably linked to said gRNA;
b) a second regulatory element operably linked to the base editor;
wherein components a) and b) are located on the same vector or on separate vectors;
optionally, said vector is a vector for targeted editing of the AGT gene;
optionally, said vector comprises a plasmid or a viral vector;
optionally, said viral vector is selected from the group consisting of adeno-associated virus (AAV), adenovirus, lentivirus, retrovirus, herpes virus, SV40, poxvirus, and combinations thereof.

4. A gene editing system, wherein said gene editing system comprises the gRNA of claim 1;
optionally, said gene editing system further comprises a base editor;
optionally, said base editor is a base editor fused to a nuclease;
optionally, said nuclease comprises a Cas protein;
optionally, said nuclease comprises a Type II Cas protein or a Type V Cas protein;
optionally, said nuclease comprises Cas9, CasX, CasY, Cpf1, C2c1, C2c2, C2c3, Cas12a, Cas12b, Cas12g, Cas12h, Cas12i, Cas13b, Cas13c, Cas13d, Cas14, or Argonaute (Ago);
optionally, said base editor comprises an adenine base editor (ABE);
optionally, said base editor comprises ABE8e;
optionally, said gene editing comprises *in vivo* gene editing or *in vitro* gene editing.

5. A cell, wherein said cell is obtained by editing it with the gene editing system of claim 4;
optionally, said cell is an in vitro cultured cell;
optionally, said cell comprises a primary cell or a passaged cell;
optionally, said cell comprises a mammalian cell;
optionally, said cell comprises a human cell;
optionally, said cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

6. A composition comprising a gRNA component and a protein component, wherein said gRNA component comprises the gRNA of claim 1, and said protein component comprises a base editor;
optionally, said gRNA component is selected from the group consisting of the gRNA of claim 1 and a nucleic acid encoding said gRNA; said base editor is selected from the group consisting of a base editor and a nucleic acid encoding the base editor;
optionally, said base editor is a base editor fused to a nuclease;
optionally, said nuclease comprises a Cas protein;
optionally, said nuclease comprises a Type II Cas protein or a Type V Cas protein;
optionally, said nuclease comprises Cas9, CasX, CasY, Cpf1, C2c1, C2c2, C2c3, Cas12a, Cas12b, Cas12g, Cas12h, Cas12i, Cas13b, Cas13c, Cas13d, Cas14, or Argonaute (Ago);
optionally, said base editor comprises an adenine base editor;
optionally, said base editor comprises ABE8e;
optionally, said composition comprises a pharmaceutical composition;
optionally, said pharmaceutical composition is in a liquid form;
optionally, the dosage form of said pharmaceutical composition comprises an injection;
optionally, the dosage form of said pharmaceutical composition is an intravenous injection.

7. A composition comprising:
the system of claim 4 or the cell of claim 5; and
a pharmaceutically acceptable carrier;
optionally, wherein in said composition, said system or said cell accounts for 1 to 99 wt%, preferably 10 to 90 wt%, more preferably 30 to 70 wt% of the total weight of the composition.

8. A delivery composition comprising an active ingredient and a delivery vehicle, wherein said active ingredient comprises the gRNA of claim 1, the vector of claim 3, or the gene editing system of claim 4;
optionally, said delivery composition further comprises a base editor;
optionally, said delivery vehicle comprises lipid particles, sugar particles, metal particles, protein particles, liposomes, exosomes, microvesicles, gene guns, or viral vectors such as replication-defective retroviruses, lentiviruses, adenoviruses, or adeno-associated viruses.

9. A host cell, wherein said host cell comprises the gRNA of claim 1, the nucleic acid of claim 2, the vector of claim 3, the gene editing system of claim 4, the composition of claim 6, or the delivery composition of claim 8;
optionally, said host cell comprises a mammalian cell;
optionally, said host cell comprises a human cell;
optionally, said host cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

10. A CRISPR complex, wherein said complex comprises the gRNA of claim 1, a base editor, and a target nucleic acid bound to said gRNA.

11. A kit comprising:
a first container, and an active ingredient or a medicament containing said active ingredient, wherein said active ingredient or said medicament is contained in the first container; said active ingredient comprises the gRNA of claim 1, the nucleic acid of claim 2, the vector of claim 3, the gene editing system of claim 4, the cell of claim 5, the composition of claim 6 or 7, the delivery composition of claim 8, the host cell of claim 9, or the CRISPR complex of claim 10;
optionally, said active ingredient or said medicament is a single-active ingredient preparation;
optionally, the dosage form of said active ingredient or said medicament is an injection;
optionally, the dosage form of said active ingredient or said medicament is an intravenous injection;
optionally, said kit further comprises instructions of administering said active ingredient or said medicament to a subject in need thereof, so as to (i) increase gene editing efficiency; and/or (ii) prevent and/or treat a disease;
optionally, said disorder or disease comprises an angiotensinogen-related disease;
optionally, said angiotensinogen-related disease is selected from the group consisting of hypertension, borderline hypertension, essential hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atheroma, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary aldosteronism and other mineralocorticoid excess states, sleep apnea, thyroid/parathyroid disorders, heart failure, myocardial infarction, angina pectoris, stroke, diabetes mellitus, nephropathy, renal failure, systemic sclerosis, intrauterine growth restriction (IUGR), and fetal growth restriction;
optionally, said angiotensinogen-related disease is selected from the group consisting of hypertension, hypertensive heart disease, hypertensive nephropathy, pregnancy-associated hypertension, atheroma, arteriosclerosis, chronic kidney disease, glomerulosclerosis, aortic coarctation, aortic aneurysm, ventricular fibrosis, Cushing's syndrome and other glucocorticoid excess states (including chronic steroid therapy), pheochromocytoma, primary aldosteronism and other mineralocorticoid excess states, sleep apnea, thyroid/parathyroid disorders, heart failure, myocardial infarction, stroke, diabetes mellitus, renal failure, and systemic sclerosis;
optionally, said angiotensinogen-related disease is pregnancy-associated hypertension (e.g., pregnancy-induced hypertension, preeclampsia, and eclampsia);
optionally, said disorder or disease is hypertension;
optionally, said subject of administration is a cell;
optionally, said subject of administration is a human cell;
optionally, said subject of administration comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell;
optionally, said subject of administration is a human or a non-human mammal;
optionally, said administration comprises contacting said active ingredient or said medicament with a human hepatocyte, or injecting said active ingredient or said medicament into a human body.

12. A method for gene editing a cell, comprising contacting said cell with the gRNA of claim 1, the nucleic acid of claim 2, the vector of claim 3, the gene editing system of claim 4, the composition of claim 6 or 7, the delivery composition of claim 8, the host cell of claim 9, the CRISPR complex of claim 10, or the kit of claim 11;
optionally, said contacting is performed in vitro;
optionally, said cell is an in vitro cultured cell;
optionally, said cell comprises a primary cell or a passaged cell;
optionally, said cell comprises a mammalian cell;
optionally, said cell comprises a human cell;
optionally, said cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

13. A kit for gene editing, wherein said kit comprises the gRNA of claim 1, the nucleic acid of claim 2, the vector of claim 3, the gene editing system of claim 4, the composition of claim 6 or 7, the delivery composition of claim 8, the host cell of claim 9, the CRISPR complex of claim 10, or the kit of claim 11;
optionally, said kit further comprises a label or instructions;
optionally, said label or said instructions show instructions of administering the gRNA of claim 1, the nucleic acid of claim 2, the vector of claim 3, the system of claim 4, the composition of claim 6 or 7, the delivery composition of claim 8, the host cell of claim 9, the CRISPR complex of claim 10, or the kit of claim 11 to a subject to be edited, so as to perform gene editing;
optionally, said subject to be edited comprises a cell;
optionally, said cell comprises a primary cell or a passaged cell;
optionally, said cell comprises a mammalian cell;
optionally, said cell comprises a human cell;
optionally, said cell comprises a hepatocyte, a basal ganglia cell, a brain cell, a cardiomyocyte, a nerve cell, a gallbladder cell, a kidney cell, a skeletal muscle cell, a choroid plexus cell, a retinal cell, a pituitary cell, a prostate cell, an adrenal cell, a thyroid cell, an adipocyte, an intestinal cell, a cervical cell, or a pancreatic cell.

14. Use of the gRNA of claim 1, the nucleic acid of claim 2, the vector of claim 3, the gene editing system of claim 4, the composition of claim 6 or 7, the delivery composition of claim 8, the host cell of claim 9, the CRISPR complex of claim 10, the kit of claim 11, or the kit of claim 13 in gene editing, gene targeting, or gene cleavage.

15. Use of the gRNA of claim 1, the nucleic acid of claim 2, the vector of claim 3, the system of claim 4, the cell of claim 5, the composition of claim 6 or 7, the delivery composition of claim 8, the host cell of claim 9, the CRISPR complex of claim 10, the kit of claim 11, or the kit of claim 13 in the manufacture of a medicament for prevention and/or treatment of a disease;
optionally, said disease is a disorder caused by a defect in a target sequence at a target locus (e.g., the AGT gene);
optionally, said disorder or disease comprises an angiotensinogen-related disease;
optionally, said angiotensinogen-related disease is selected from the group consisting of hypertension, borderline hypertension, essential hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atheroma, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary aldosteronism and other mineralocorticoid excess states, sleep apnea, thyroid/parathyroid disorders, heart failure, myocardial infarction, angina pectoris, stroke, diabetes mellitus, nephropathy, renal failure, systemic sclerosis, intrauterine growth restriction (IUGR), and fetal growth restriction;
optionally, said angiotensinogen-related disease is selected from the group consisting of hypertension, hypertensive heart disease, hypertensive nephropathy, pregnancy-associated hypertension, atheroma, arteriosclerosis, chronic kidney disease, glomerulosclerosis, aortic coarctation, aortic aneurysm, ventricular fibrosis, Cushing's syndrome and other glucocorticoid excess states (including chronic steroid therapy), pheochromocytoma, primary aldosteronism and other mineralocorticoid excess states, sleep apnea, thyroid/parathyroid disorders, heart failure, myocardial infarction, stroke, diabetes mellitus, renal failure, and systemic sclerosis;
optionally, said angiotensinogen-related disease is pregnancy-associated hypertension (e.g., pregnancy-induced hypertension, preeclampsia, and eclampsia);
optionally, said disorder or disease is hypertension.

16. A method for treating a disease, comprising administering to a subject an effective amount of the gene editing system of claim 4, the cell of claim 5, the composition of claim 6 or 7, the delivery composition of claim 8, the host cell of claim 9, the CRISPR complex of claim 10, the kit of claim 11, or the kit of claim 13; wherein
optionally, said administration comprises administration by injection;
optionally, said subject is a human or a non-human mammal;
optionally, said non-human mammal comprises rodents and primates, preferably mice, rats, rabbits, or monkeys;
optionally, said disease is a disorder caused by a defect in a target sequence at a target locus (e.g., the AGT gene);
optionally, said disorder or disease comprises an angiotensinogen-related disease;
optionally, said angiotensinogen-related disease is selected from the group consisting of hypertension, borderline hypertension, essential hypertension, secondary hypertension, hypertensive crisis, hypertensive urgency, isolated systolic and diastolic hypertension, pregnancy-associated hypertension, diabetic hypertension, resistant hypertension, refractory hypertension, paroxysmal hypertension, renovascular hypertension, Goldblatt hypertension, ocular hypertension, glaucoma, pulmonary hypertension, portal hypertension, systemic venous hypertension, systolic hypertension, labile hypertension; hypertensive heart disease, hypertensive nephropathy, atheroma, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, Cushing's syndrome and other glucocorticoid excess states (including chronic steroid therapy), pheochromocytoma, reninoma, secondary aldosteronism and other mineralocorticoid excess states, sleep apnea, thyroid/parathyroid disorders, heart failure, myocardial infarction, angina pectoris, stroke, diabetes mellitus, nephropathy, renal failure, systemic sclerosis, intrauterine growth restriction (IUGR), and fetal growth restriction;
optionally, said angiotensinogen-related disease is selected from the group consisting of hypertension, hypertensive heart disease, hypertensive nephropathy, pregnancy-associated hypertension, atheroma, arteriosclerosis, chronic kidney disease, glomerulosclerosis, aortic coarctation, aortic aneurysm, ventricular fibrosis, Cushing's syndrome and other glucocorticoid excess states (including chronic steroid therapy), pheochromocytoma, primary aldosteronism and other mineralocorticoid excess states, sleep apnea, thyroid/parathyroid disorders, heart failure, myocardial infarction, stroke, diabetes mellitus, renal failure, and systemic sclerosis;
optionally, said angiotensinogen-related disease is pregnancy-associated hypertension (e.g., pregnancy-induced hypertension, preeclampsia, and eclampsia);
optionally, said disorder or disease is hypertension.
